# EUROPEAN PATENT APPLICATION

(11) **EP 1 541 678 A1**
(43) Date of publication of application: **15.06.2005**
(21) Application number: 03792844.7
(22) Date of filing: 26.08.2003
(51) Int. Cl.: C12N 15/00, C12M 1/00, C07H 21/00, G01N 33/50

(54) **NUCLEIC ACID ANALYSIS CHIP AND NUCLEIC ACID ANALYZER**

(30) Priority: 26.08.2002 JP 2002245900
(71) Applicant: Japan Science and Technology Agency, Kawaguchi-shi, Saitama 332-0012 (JP)
(72) Inventor: YASUDA, Kenji, Koto-ku, Tokyo 135-0052 (JP)
(74) Representative: Calamita, Roberto
(86) International application number: PCT/JP2003/010762
(87) International publication number: WO 2004/018664

(57) **Abstract**

Means for performing direct quantitative analysis of the components of nucleic acids of cell 103 placed in a well as culture container (102) of a cell culture microchamber, comprising means for optically measuring each of the culture containers (102) of cell culture microchamber, means for effecting optical heating, means for introducing a reaction fluid in each of the culture containers (102) of cell culture microchamber and means for inhibiting diffusion and evaporation of the reaction fluid in each of the culture containers (102). These enable analyzing the components of nucleic acids of each of the cells cultured in single-cell culture microchamber.

## Description

### Technical Field

The present invention relates to a nucleic acid analysis chip and a nucleic acid analyzer using the same. More specifically, the present invention relates to a novel nucleic acid analysis chip for analyzing the components of nucleic acids of a cell cultured in a cell culturing micro chamber for culturing per one cell unit while observing a specific cell with a microscope in the biotechnology research field using microorganisms and cells, and an analyzer using the same.

### Background Art

Conventionally, in the fields of biology, medicine and pharmacology, for the observation of the state change of a cell or the response of a cell to a chemical, or the like, the average value of the values of a cell group has been treated as if it represents the characteristics of the cell. However, in reality, the cells rarely have the cell cycles synchronized in a group, and thus each cell generates a protein by different cycles. In order to solve the problems, the inventors have newly invented a technique for selecting only one specific cell and culturing the cell as a cell strain, a technique for controlling the solution environment conditions of the cell and controlling constantly the cell concentration in the container in the case of observing a cell, and a technique for culture and observation while specifying the cell interacting with each other and have filed the same as the Japanese Patent Application No. 2000-356827. Measurement of the cells per one cell unit has been enabled thereby.

### Disclosure of Invention

However, according to the micro chamber realized by the present inventor as mentioned above as a culturing technique enabling the measurement while completely controlling the environment for one cell unit, in order to measure the change of the cells such as the response to the chemical, the expressed mRNA amount in the cell should be measured quantitatively. In this regard, a nucleic acid analyzing technique optimized for one cell culturing system has not been developed in the present situation.

Then, based on the above-mentioned background, an object of the present invention is to provide a new technological means for analyzing the components of nucleic acids of each cell cultured in a cell culturing micro chamber.

In order to solve the above-mentioned problems, the present invention provides a nucleic acid analysis chip comprising a means for directly measuring the quantitative analysis of the components of nucleic acids of a cell in each container of the above-mentioned cell culturing micro chamber developed by the present inventors, and a nucleic acid analyzer.

That is, the present invention firstly provides a nucleic acid analysis chip having a plurality of holes capable of completely sealing a single cell provided as the culturing containers on a substrate, and an upper area to be filled with a liquid having a specific gravity lighter than that of an aqueous solution while leaving the aqueous solution in the holes without mixing the upper part of the holes with the aqueous solution, wherein a reaction solution can be introduced into the above-mentioned holes for executing optically the nucleic acid analysis of the cells.

Moreover, the present invention secondly provides a nucleic acid analyzer using the nucleic acid analysis chip. The analyzer comprises a means for optically measuring each container in a micro chamber, an optically heating means, a means for introducing a reaction solution to each container in the micro chamber, a means for preventing diffusion and evaporation of the reaction solution in each container, or the like.

### Brief Description of Drawings

FIG. 1 is a schematic diagram showing an example of the basic configuration of a nucleic acid analysis chip according to the present invention.
FIG. 2 is a schematic diagram showing an example of a device configuration for observing the nucleic acid analysis chip shown in FIG. 1 and heating locally by a convergent light beam.

### The numerals in the figures denote the following.

- 100: nucleic acid analysis chip
- 101: optically transparent substrate
- 102: culturing container
- 103: cell
- 104: micro pipette
- 105: upper area
- 106: objective lens
- 107: semi permeable film
- 201: light source
- 202, 209, 212: filter
- 203: condenser lens
- 204: stage with the temperature adjusting function
- 205: objective lens
- 206: movable dichroic mirror
- 208: light source
- 210: dichroic mirror
- 211: mirror
- 213: camera
- 214: image processing analyzing device
- 215: stage moving motor

### Best Mode for Carrying Out the Invention

The present invention has the above-mentioned characteristics. As to the application thereof, for example, various embodiments such as collection of components of nucleic acids from the holes after the introduction of the reaction solution and employment of a sealing material such as a semi permeable film between the holes and the upper area can be adopted for the nucleic acid analysis chip.

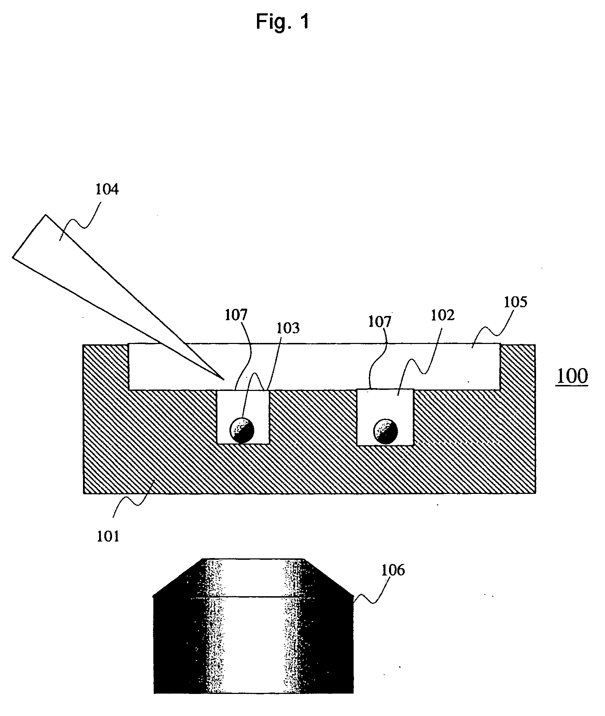

First, an example of the basic configuration of a nucleic acid analysis chip according to the present invention will be explained with reference to the embodiment of FIG. 1. The basic configuration of the nucleic acid analysis chip 100 is same as the above-mentioned cell culturing micro chamber. That is, a plurality of cell culturing containers 102 are provided on an optically transparent substrate 101 so that a cell 103 can be placed in each container 102 so as to be cultured. The upper surface of the substrate 101 is sealed with a semi permeable film 107 so that the culturing solution can be replaced and the cells can be cultured continuously without escape of the cells 103 from the containers or introduction of impurities such as bacteria. In the case of revealing the information of the components of nucleic acids in the cells while culturing, the culturing solution in the upper surface of the above-mentioned containers 102 is eliminated totally and a liquid not to be mixed with an aqueous solution and having a specific gravity lighter than water such as a fluid paraffin and a silicone oil is introduced into the part in the upper area 105. Then, the containers 102 are isolated by the liquid such as the fluid paraffin. In the state, a PCR reaction solution is introduced with the semi permeable film 107 on the upper surface of the above-mentioned containers 102 broken using a micro pipette 104. Here, for example, by adding a fluorescent pigment capable of optically measuring the PCR reaction, such as Taq Man Probe (Amersham, U. S. A.), the nucleic acid quantitative measurement utilizing the fluorescence energy moving method can be enabled. In the case the fluorescence amount is measured, the fluorescence collected by the objective lens 106 can be measured. Moreover, in order to heat the containers, a heating operation necessary of the PCR reaction can be executed by directing an infrared ray through the objective lens 106. Moreover, for the analysis of the unknown components of nucleic acids, the components of nucleic acids collected from the containers 102 by the micro pipette 104 can be analyzed comprehensively and quantitatively using the capillary electrophoresis. In this case, since the nucleic acid analysis chip needs not be heated optically, the PCR reaction can be carried out by an existing heating and cooling device such as a thermal cycler.

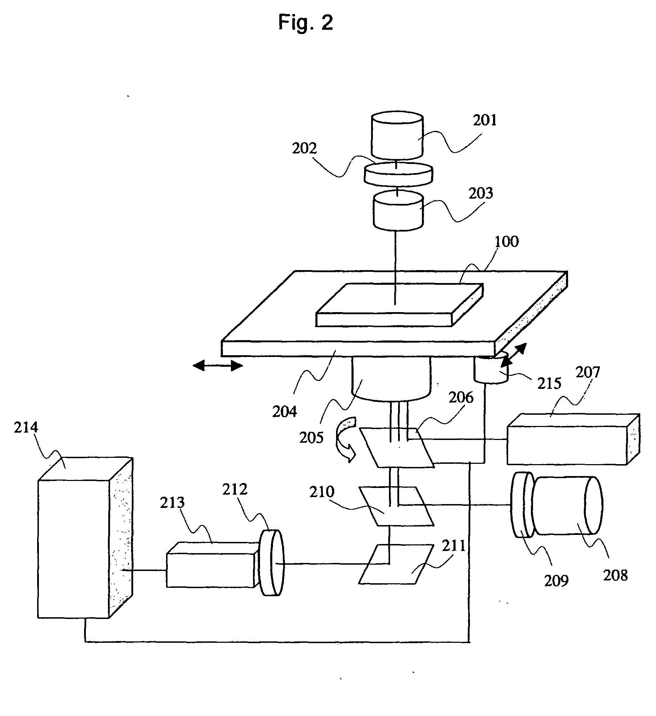

FIG. 2 shows an example of a device configuration for guiding a convergent light beam for generating the PCR reaction by heating the containers 102 in the cell culturing micro chamber 100. According to the device, a microscope observation system is provided for observing the change of the specimen such as the cell in the nucleic acid analysis chip 100 and the fluorescence amount change, and an infrared convergent light beam directing system is provided for heating the aqueous solution in the containers at the same time. As it is shown also in FIG. 2, a nucleic acid analysis chip 100 is disposed on the optical path of the microscope observation optical system. First, the microscope observation optical system has the following configuration. A light beam outputted from the light source 201 is adjusted to have a specific wavelength by the filter 202 and it is converged by the condenser lens 203 so as to be directed to the nucleic acid analysis chip 100. The directed light beam is used as the transmitted light beam for the observation with the objective lens 205. The transmitted light image inside the nucleic acid analysis chip 100 is guided to the camera 213 after passing through the filter 212 by the mirror 211 so as to be focused on the light receiving surface of the camera. Therefore, the material of the chip 100 is preferably a material optically transparent with respect to a light beam of the wavelength selected by the filter 202. Specifically, a glass such as a borosilicate glass and a quartz glass, a resin or a plastic such as a polystyrene, or a solid substrate such as a silicon substrate are used. More preferably, particularly in the case a silicon substrate is used, it is preferable to use a light beam of a 900 nm or more wavelength.

Moreover, an exciting light beam for the fluorescence observation outputted from the light source 208 is also guided to the objective lens 205 by the dichroic mirror 210 after the wavelength selection by the filter 209 so as to be used as the exciting light beam for the fluorescence observation inside the nucleic acid analysis chip 100. The fluorescence emitted from the chip 100 is collected again by the objective lens 205 so that only the fluorescence and the transmitted light beam after cutting the exciting light beam by the filter 212 can be observed by the camera 213. At the time, by adjusting the combination of the filters 202, 209, 212, observation of only the transmitted light beam by the camera 213, observation of only the fluorescence or simultaneous observation of the transmitted light beam image and the fluorescence image can be enabled. On the optical path there is provided a mechanism for guiding the laser beam generated by the laser light source 207 to the objective lens 205 by the movable dichroic mirror 206. The laser beam is processed to be a convergent light beam by the objective lens 205 so that the nucleic acid analysis chip 100 can be heated locally. In the case of moving the light focusing point, by moving the movable dichroic mirror, the laser beam converging position inside the nucleic acid analysis chip 100 can be moved. As to the laser beam wavelength, a wavelength having the water absorption but not having the optical chemical function and a little absorption to the substrate is preferable. For example, in the case of a 1,500 nm wavelength light beam, the laser beam absorption is generated effectively with water the reaction solution generates a heat locally in the container with the light beam absorbed. '

Moreover, the image data obtained by the camera are analyzed by the image processing analysis device 214 so as to drive the stage moving motor 215 for moving in the X-Y direction freely for the control of the position of the movable dichroic mirror 206 and the XY stage with the temperature adjusting function 204 with the nucleic acid analysis chip 100 placed thereon on the basis of the various results of the analysis. Moreover, it is also possible to have the PCR reaction for the entire chip by the temperature adjusting device.

Although measurement is carried out using one camera in the above-mentioned embodiment, according to the present invention, for example, the quantitative analysis can be carried out by the above-mentioned energy moving method by adding a dichroic mirror for measuring the change of the fluorescence intensity by at least two different wavelengths.

Of course it is needless to say that the present invention is not limited to the above-mentioned embodiments and various embodiments can be employed for the details of the configuration.

### Industrial Applicability

As heretofore described, according to the present invention, the components of nucleic acids of each cell cultured in a single cell culturing micro chamber can be analyzed.

## Claims

1. A nucleic acid analysis chip having a plurality of holes capable of completely sealing a single cell provided as the culturing containers on a substrate, and an upper area to be filled with a liquid having a specific gravity lighter than that of an aqueous solution while leaving the aqueous solution in the holes without mixing the upper part of the holes with the aqueous solution, **characterized in that** a reaction solution can be introduced into the above-mentioned holes for executing optically the nucleic acid analysis of the cells.

2. The nucleic acid analysis chip according to claim 1, **characterized in that** components of nucleic acids can be collected from the holes after the introduction of the reaction solution.

3. The nucleic acid analysis chip according to claim 1 or 2, **characterized in that** a sealing material capable of introducing the reaction solution to the holes and furthermore collecting the components of nucleic acids from the holes is provided between the holes as the culturing containers and the upper area.

4. The nucleic acid analysis chip according to claim 3, **characterized in that** the sealing material is a semi permeable film capable of exchanging the culturing solution.

5. A nucleic acid analyzer using the nucleic acid analysis chip according to any of claims 1 to 4, **characterized in** comprising a means for optically measuring a reaction in the holes as the culturing containers.

6. The nucleic acid analyzer according to claim 5, **characterized in** comprising a means for measuring the change of the fluorescence amount of a specific wavelength.

7. The nucleic acid analyzer according to claim 5 or 6, **characterized in** comprising a means for directing a convergent light beam of a wavelength having the water absorption, capable of heating the aqueous solution in the containers.

8. The nucleic acid analyzer according to any of claims 5 to 7, **characterized in** comprising a means for introducing a reaction solution to the holes and a means for extracting the components of nucleic acids from the holes.
